# EUROPÄISCHE PATENTSCHRIFT

(11) **EP 3 184 642 B1**
(45) Veröffentlichungstag und Bekanntmachung des Hinweises auf die Patenterteilung: **08.05.2019**
(21) Anmeldenummer: 15202233.1
(22) Anmeldetag: 22.12.2015
(51) Int. Cl.: C12N 1/19, C12N 15/81, C12P 21/02, C12R 1/84

(54) **HEFEZELLE**
YEAST CELL
CELLULE DE LEVURE

(43) Veröffentlichungstag der Anmeldung: 28.06.2017
(73) Patentinhaber: bisy e.U., 8200 Hofstätten/Raab (AT)
(72) Erfinder: Vogl, Thomas, 8052 Graz (AT)
(74) Vertreter: Schwarz & Partner Patentanwälte OG

(56) Entgegenhaltungen:
- EP-A1- 0 242 007
- WO-A1-03/095653
- RASCHKE W C ET AL: "Inducible expression of a heterologous protein in Hansenula polymorpha using the alcohol oxidase 1 promoter of Pichia pastoris", GENE, ELSEVIER, AMSTERDAM, NL, Bd. 177, Nr. 1, 24. Oktober 1996 (1996-10-24), Seiten 163-167, XP004043391, ISSN: 0378-1119, DOI: 10.1016/0378-1119(96)00293-4
- HARTNER FRANZ S ET AL: "Regulation of methanol utilisation pathway genes in yeasts", MICROBIAL CELL FACTORIES, BIOMED CENTRAL, Bd. 5, Nr. 1, 14. Dezember 2006 (2006-12-14), Seite 39, XP021023935, ISSN: 1475-2859, DOI: 10.1186/1475-2859-5-39
- FRANZ S HARTNER ET AL: "Promoter library designed for fine-tuned gene expression in Pichia pastoris", NUCLEIC ACIDS RESEARCH, OXFORD UNIVERSITY PRESS, GB, Bd. 36, Nr. 12, 1. Juli 2008 (2008-07-01), Seiten e76-1, XP002733013, ISSN: 1362-4962, DOI: 10.1093/NAR/GKN369 [gefunden am 2008-06-06]
- KATRIN WEINHANDL ET AL: "Carbon source dependent promoters in yeasts", MICROBIAL CELL FACTORIES, BIOMED CENTRAL, Bd. 13, Nr. 1, 9. Januar 2014 (2014-01-09) , Seite 5, XP021172491, ISSN: 1475-2859, DOI: 10.1186/1475-2859-13-5
- GELLISSEN G ET AL: "New yeast expression platforms based on methylotrophic Hansenula polymorpha and Pichia pastoris and on dimorphic Arxula adeninivorans and Yarrowia lipolytica - A comparison", FEMS YEAST RESEARCH, WILEY-BLACKWELL PUBLISHING LTD, GB, NL, Bd. 5, Nr. 11, 1. November 2005 (2005-11-01), Seiten 1079-1096, XP027680530, ISSN: 1567-1356 [gefunden am 2005-11-01]

## Beschreibung

Die vorliegende Erfindung betrifft die Verwendung orthologer Promotoren in Hefezellen.

Rekombinante Proteine wie Biopharmazeutika oder industriell relevante Biokatalysatoren werden am häufigsten mittels heterologer Genexpression in Mikroorganismen hergestellt. *Escherichia coli, Saccharomyces cerevisiae* und filamentöse Pilze werden seit jeher häufig für die rekombinante Proteinproduktion verwendet. In den letzten zwei Jahrzehnten haben sich die methylotrophen Hefen *Komagataella (Pichia) pastoris, Komagataella (Pichia) phaffii(Pp), Komagataella Kurtzmanii, Ogataea (Hansenula) polymorpha* (Hp), *Candida boidinii* (Cb) und *Ogataea (Pichia) methanolica* (Pm) als leistungsfähige alternative Produktionsstämme etabliert. Diese Stämme ermöglichen es bei hoher Zelldichte hohe Expressionsraten für heterologe Proteine zu erzielen. Von den zuvor genannten vier Hefearten, wird *P. pastoris* (*Komagataella phaffii*) mittlerweile am häufigsten für die heterologe Proteinproduktion eingesetzt.

Alle methylotrophen Hefen weisen streng regulierte, starke Promotoren auf, die bei der Regulation der Expression von Genen der Methanolverwertung ("methanol utilization"; MUT) beteiligt sind. Üblicherweise werden Promotoren von Genen der Methanolverwertung auf reprimierenden Kohlenstoffquellen, wie Glukose, reprimiert und in Anwesenheit von Methanol als Kohlenstoffquelle stark hochreguliert. Ist die reprimierende Kohlenstoffquelle erschöpft oder in Anwesenheit einer nicht-reprimierenden Kohlenstoffquelle, kommt es zur Aktivierung des Promoters durch Derepression, wobei die Stärke dieser Wirkung allerdings zwischen Spezies und sogar innerhalb des gleichen Organismus beträchtlich variieren kann. Der Promotor des Alkoholoxidase-1-Gens in P. *pastoris* GS115(PPpAOX1), beispielsweise, weist unter dereprimierenden Bedingungen im Vergleich zu Methanol induzierten Bedingungen nur eine 2-4%ige Aktivität auf. Im Gegensatz dazu zeigt der Promotor des orthologen Gens (Methanol-Oxidase, MOX) in *H*. *polymorpha* (PHpMOX) unter dereprimierenden Bedingungen eine Aktivität im Vergleich zu Methanol induzierten Bedingungen von bis zu 70% auf. Auch die Promotoren der orthologen Gene in *C*. *boidinii* (Alkoholoxidase 1, "AOD1") und *P. methanolica* (Methanoloxidase 1/2, "MOD1/2") zeigen ein vergleichbares Verhalten.

In der EP 0 242 007 wird ein Verfahren zur Herstellung einer Oxidase oder Oxidase umfassenden Zusammensetzung mittels Hefezellen beschrieben. Die Expression der Oxidase kann mit Hilfe eines Methanoloxidase (MOX) Promoters gesteuert werden.

Hartner FS et al. (Nucleic Acids Res 36(2008) :e76) beschreibt eine AOX1 Promoter Bibliothek in *Pichia pastoris.*

Weinhandl K et al. (Microb Cell Fact 13(2014):5) ist ein Übersichtsartikel über Kohlenstoffquelle abhängige Promotoren in Hefen.

Gellissen G et al. (FEMS Yeast Res 5(2005) :1079-1096) beschäftigen sich mit Expressionssystemen wie *Hansenula polymorpha* und *Pichia pastoris* und vergleichen diese.

In der WO 03/095653 werden FMD Promotorvarianten beschrieben, die unter dereprimierenden Bedingungen eine im Vergleich zum Wildtyp-Promoter signifikant höhere Proteinexpressionsaktivität aufweisen.

Die Expressionsinduktion mit toxischem und brennbarem Methanol ist besonders im großtechnischen Maßstab aus Gründen der Betriebssicherheit unerwünscht, so dass starke dereprimierte Promotoren eine günstige Alternative darstellen. Dementsprechend wurden in letzter Zeit PPpAOX1 Varianten, alternative Promotoren und neuartige MUT Promotoren mit unterschiedlichen dereprimierenden Eigenschaften entwickelt, um eine Methanol-freie Proteinexpression im Industriemaßstab zu ermöglichen. Da die Expressionsraten derartiger Promotoren im Vergleich zu Methanolinduzierte Promotoren in der Regel weitaus geringer ist, ist es eine Aufgabe der vorliegenden Erfindung alternative Möglichkeiten zur induzierbaren und starken Methanol-freien Überexpression von rekombinanten Proteinen in Hefen, wie *P. pastoris,* zur Verfügung zu stellen.

Dieses Ziel wird mit einer *Pichia pastoris* Zelle umfassend einen orthologen durch Derepression induzierbaren Promoter einer methylotrophen Hefezelle oder eine durch Derepression induzierbare Variante davon erreicht, bei der der orthologe Promoter in der methylotrophen Hefezelle in der Lage ist, die Expression von Polypeptiden unter dereprimierenden Bedingungen zu steuern, wobei der orthologe Promoter ein orthologer Formiatdehydrogenase (FMD) Promoter einer methylotrophen Hefezelle und die Variante mindestens 90% ident mit dem Promoter ist, wobei der orthologe Promoter eine Nukleinsäuresequenz SEQ ID Nr. 1 aufweist und operativ an ein Nukleinsäuremolekül kodierend für ein heterologes oder homologes Polypeptid gebunden ist und das ursprüngliche Regulationsprofil des orthologen Promoters in Hefezellen der Gattung Komagataella beibehalten wird.

Es hat sich überraschender Weise gezeigt, dass Promotoren, die in der Lage sind, in anderen methylotrophen Hefezellen, die vorzugsweise nicht der Gattung Komagataella (Pichia) zugehören, die Expression von Polypeptiden unter dereprimierenden Bedingungen zu steuern (z.B. im Vergleich zu nicht-dereprimierenden Bedingungen zu erhöhen), auch in Hefezellen der Gattung Komagataella (Pichia) vergleichbare Eigenschaften aufweisen.

Zudem hat sich überraschender Weise gezeigt, dass ein Formiatdehydrogenase (FMD) Promoter einer methylotrophen Hefezelle, der natürlicherweise nicht in einer Hefezelle der Gattung Komagataella bzw. in derselben Hefezelle vorkommt, in eben dieser Zelle besondere Eigenschaften aufweist. Ein orthologer FMD Promoter ist in Komagataella Zellen sowohl unter dereprimierenden Bedingungen als auch unter Methanol-induzierten Bedingungen signifikant stärker als alle bisher getesteten natürlich in Komagataella vorkommenden Promotoren, die in der Regulation der Expression von Genen der Methanolverwertung ("MUT Promotoren") beteiligt sind. So ist ein orthologer FMD Promoter unter dereprimierenden Bedingung signifikant stärker als die natürlich in Komagataella Zellen vorkommenden Promoteren CAT1 und GAP, beispielsweise. Überraschenderweise sind orthologe FMD Promotoren bereits unter den verwendeten Screening Bedingungen unter dereprminierenden Bedingungen sogar ähnlich stark als die natürlicherweise in Komagataella vorkommenden AOX (AOX1 und AOX2) Promotoren unter Methanol-induzierenden Bedingungen. Solche Effekte lassen sich üblicherweise unter kontrollierten C-Quellendosierungen im Bioreaktorversuch deutlich verstärken. Dies bedeutet, dass orthologe FMD Promotoren die üblicherweise in Komagataella verwendeten AOX Promotoren ersetzen können. Dabei können im Wesentlichen idente oder sogar höhere Protein-Expressionsausbeuten erzielt werden wie bei herkömmlichen Methanol-induzierten Expressionssystemen ohne jedoch Methanol als Induktionsmittel zu verwenden. Dabei ist es überraschend, dass ein Formiatdehydrogenase (FMD) Promoter einer methylotrophen Hefezelle (z.B. von *H. polymorpha*), der auch in dieser Hefezelle (z.B. in *H. polymorpha*) signifikant dereprimiert ist, dieses Regulationsprofil auch in einer anderen methylotrophen Hefezelle (z.B. *P. pastoris*) beibehält. Frühere Studien haben im Gegensatz dazu gezeigt, dass bei einem Transfer von Promotoren zwischen methylotrophen Hefen das Regulationsprofil des fremden Promoters nicht übertragen wird (z.B. ist der *P. pastoris* AOX1 Promotor beispielsweise in *H. polymorpha* nicht stringent reprimiert wie natürlicherweise in *P. pastoris;* siehe z.B. Raschke WC et al. Gene 177(1996) :163-167 und Rodriguez L et al. Yeast 12(1996) :815-822.). Entsprechend ist die gängige Meinung in der Fachwelt, dass unterschiedliche Regulation zwischen methylotrophen Hefezellen nicht durch die Promotersequenz sondern durch unterschiedliche Regualtionsmechanismen in den Hefezellen zustande kommen (siehe z.B. Hartner FS et al. Microb Cell Fact 5 (2006) :39-59). Überraschenderweise hat sich aber gezeigt, dass die starke Aktivierung eines Formiatdehydrogenase (FMD) Promoter einer methylotrophen Hefezelle (z.B. von *H. polymorpha*) durch Derepression nicht nur auf andere methylotrophe Hefezellen, wie z.B. *Komagataella phaffii,* übertragbar ist, sondern sogar die technischen Eigenschaften der starken homologer Promotoren wie der des AOX1 Gens und des CAT1 Gens übertrifft.

Die Verwendung orthologer Promotersequenzen hat auch weitere technische Vorteile. Beispielsweise wird durch deren Verwendung die Möglichkeit zur homologen Rekombination reduziert, wodurch sich eine höhere genetische Stabilität der Expressionsstämme ergibt.

"Hefezelle der Gattung Komagataella" umfasst sämtliche Hefezellen dieser Gattung wie u.a. *Komagataella kurtzmanii, Komagataella pastoris, Komagataella phaffii, Komagataella populi, Komagataella pseudopastoris, Komagataella ulmi* und *Komagataella* sp. 11-1192. "Hefezellen der Gattung Komagataella" umfassen natürlich aus spezifische Stämme der zuvor genannten Gattungen wie z.B. *Komagataella pastoris* GS115, X-33, KM71, KM71H, CBS7435, oder NRLL Y11430, CBS704, BG10, BG11 bzw. weitere Derivate dieser Stämme.

Der Begriff "ortholog", wie hier verwendet, bezieht sich auf Nuklein- oder Aminosäuremoleküle aus unterschiedlichen Arten/Spezies, welche zumindest eine funktionelle Homologie zu entsprechenden Nuklein- und Aminosäuremolekülen anderer Spezies aufweist. "Orthologe" stammen aus unterschiedlichen Organismen, die durch Artbildung entstanden sind und die auch von einem gemeinsamen Vorfahrengen abstammen. Die Sequenzen der "Orthologe" können mitunter signifikant variieren, jedoch ist die biologische bzw. biochemische Funktion davon in der Regel nicht betroffen (z.B. AOX aus *Komagataella pastoris* ist ortholog zu MOX *aus-Hansenula polymorpha* und umgekehrt, FMD aus *Hansenula polymorpha* ist ortholog zu FDH1 in *Komagataella pastoris* und umgekehrt).

"Promoter", wie hier verwendet, umfasst mindestens eine Transkriptionsstartseite ("transcription initiation start site"), eine Bindungsstelle für einen Nukleinsäurepolymerasekomplex und zusätzliche Nukleotide, so dass diese beiden Elemente funktionell aktiv sein können und das ursprüngliche Regulationsprofil der Ausgangszelle des orthologen Promoters in Hefezellen der Gattung Komagataella beibehalten. Diese zusätzlichen Nukleotide können beispielsweise Transkriptionsfaktorbindungsstellen ("transcription factor binding sites") ausbilden. Ein "durch Derepression induzierbarer Promoter" ist ein Promoter, der unter dereprimierenden Bedingungen (siehe unten) aktiviert wird, wodurch operativ daran gebundene Nukleinsäuremoleküle kodierend für heterologe oder homologe Polypeptide transkribiert werden.

Der erfindungsgemäße orthologe FMD Promoter umfasst vorzugsweise zwischen 50 und 2000, noch mehr bevorzugt zwischen 100 und 1000, noch mehr bevorzugt zwischen 150 und 800, Nukleotide aus dem Bereich vor dem Startcodon (stromaufwärts vom 5'-Ende) der für ein Protein/Polypeptid kodierenden Region des entsprechenden den Promoter umfassenden Gens, vorzugsweise der für FMD kodierenden Region des FMD Gens, welcher 1 bis 1000, vorzugsweise 1 bis 900, noch mehr bevorzugt 1 bis 800, Nukleotide umfassen kann. Besonders bevorzugt umfasst der orthologe Promoter, vorzugsweise der orthologe FMD Promoter, die Nukleotide 1 bis 1000, vorzugsweise 1 bis 900, noch mehr bevorzugt 1 bis 800, stromaufwärts vom 5'-Ende der für das Polypeptid kodierenden Region des Gens, vorzugsweise der für FMD MOX kodierenden Region des FMD MOX Gens.

"Varianten" des orthologen Promoters, vorzugsweise des orthologen Formiatdehydrogenase (FMD) Promoters, umfassen Nukleinsäuremoleküle, die sich in einem oder mehreren (beispielsweise 2, 3, 4, 5, 10, 15, 20, 25, 50) Nukleotiden von den natürlicherweise vorkommenden orthologen Promotoren, vorzugsweise der orthologen FMD bzw. MOX Promotoren, unterscheiden. Derartige Promotervarianten sind zumindest 80%, vorzugsweise mindestens 90%, noch mehr bevorzugt mindestens 95%, noch mehr bevorzugte mindestens 98%, ident mit dem korrespondierenden Bereichen der natürlicherweise vorkommenden Promotoren.

Die erfindungsgemäß einsetzbaren Varianten von orthologen Promotoren können im Vergleich zu den natürlicherweise vorkommenden Promotoren, vorzugsweise FMD Promotoren, Deletionen, Substitutionen und Insertionen aufweisen. Die Varianten der Promotoren weisen ebenfalls die Eigenschaft auf, die Expression von Proteinen unter derepremierenden Bedingungen zu ermöglichen. Vorzugsweise werden Varianten eingesetzt, die in der Lage sind unter dereprimierenden Bedingungen mindestens 50%, vorzugsweise mindestens 60%, noch mehr bevorzugt mindestens 70%, noch mehr bevorzugt mindestens 80%, noch mehr bevorzugt mindestens 90%, noch mehr bevorzugt mindestens 100%, noch mehr bevorzugt mindestens 120%, noch mehr bevorzugt mindestens 150%, der Proteinmenge zu exprimieren, die eine *Pichia pastoris* Zelle umfassend einen natürlicherweise vorkommenden orthologen Promoter, vorzugsweise einen orthologen FMD Promoter, exprimieren würde.

Verfahren zur Identifizierung und Herstellung von Promotervarianten sind hinreichend bekannt. Üblicherweise werden Mutationen in den Promoter eingeführt, woraufhin getestet wird, ob und wie sich die Eigenschaften (z.B. Expressionsrate eines Modellproteins) der Promotervariante geändert hat.

"Varianten" des orthologen FMD Promoters umfassen auch Promotorvarianten, die die regulatorischen Elemente des natürlicherweise vorkommenden orthologen Promoters und einen alternativen Minimalpromoter bzw. Core-Promoter umfassen. Der Minimalpromoter bzw. Core-Promoter ist der Teil eines Promotors, der nur diejenigen allgemeinen Promotorelemente enthält, welche notwendig für die Transkription sind (TATA Box und Transkriptionsstart). Daher umfassen die regulatorischen Elemente der erfindungsgemäßen Varianten der orthologen Promotoren zwischen 50 und 2000, noch mehr bevorzugt zwischen 100 und 1000, noch mehr bevorzugt zwischen 150 und 800, Nukleotide aus dem Bereich vor dem Startcodon (stromaufwärts vom 5'-Ende) ohne 20 bis 100, vorzugsweise ohne 25 bis 80, noch mehr bevorzugt ohne 30 bis 70, Nukleotide unmittelbar vor dem Startpunkt der Transkription.

"Identität" bzw. "ident" bezeichnet den Grad der Übereinstimmung zwischen zwei oder mehr Nuklein- bzw. Aminosäuresequenzen, der durch die Übereinstimmung zwischen den Sequenzen bestimmt werden kann. Der Prozentsatz der "Identität" ergibt sich aus dem Prozentsatz identischer Bereiche in zwei oder mehr Sequenzen unter Berücksichtigung von Lücken oder anderen Sequenzbesonderheiten (d.h. % Identität ist die Anzahl der identischen Positionen/Gesamtanzahl der Positionen x 100). Ein besonders bevorzugtes Verfahren zur Bestimmung der Identität ist das BLAST X Programm des National Centre for Biotechnology Information (NCBI) (siehe u.a. Altschul S et al. J Mol Biol 215(1990):403-410). Dabei wird bevorzugter Weise der BLOSUM62 Algorithmus mit den Parametern "Gap" "Existence" :11 und "Extension" :1 verwendet.

Unter dem Begriff "methylotrophe Hefezellen", wie hier verwendet, werden Hefezellen subsummiert, die in der Lage sind auf Nährmedien wachsen, welche als Kohlenstoffquelle Verbindungen mit nur einem Kohlenstoffatom, z.B. Methanol, enthalten.

"Dereprimierende Bedingungen", wie bei der Kultivierung der erfindungsgemäßen Hefezellen angewandt, bedeutet, dass die Hefezellen zunächst in Anwesenheit einer reprimierenden Kohlenstoffquelle (z.B. Glukose) kultiviert werden, bis dieser grossteils oder gänzlich verbraucht wurde. Nach Reduktion der Konzentration der reprimierenden Kohlenstoffquelle (z.B. Glukose) befinden sich die Zellen in Hinblick auf die reprimierende Kohlenstoffquelle bzw. der Glukose in dereprimierenden Bedingungen wieder. Die Stärke der Repressionseffekte können abhängig von der Art der Kohlenstoffquelle sein.

Gemäß einer bevorzugten Ausführungsform der vorliegenden Erfindung ist der orthologe FMD Promoter, operativ an ein Nukleinsäuremolekül kodierend für ein heterologes oder homologes Polypeptid gebunden.

Der orthologe FMD Promoter ist operativ an ein Nukleinsäuremolekül kodierend für ein heterologes (nicht von Komagataella stammend) oder homologes Polypeptid (von Komagataella stammend) gebunden sein und somit die Expression dieses Polypeptids beeinflussen bzw. steuern. Das zu regulierende Polypeptid umfasst mindestens 5, vorzugsweise mindestens 10, noch mehr bevorzugt mindestens 50, Aminosäurereste und umfasst somit Moleküle, die auch als Peptide oder Proteine bezeichnet werden.

Besonders bevorzugt kodiert das Nukleinsäuremolekül für Polypeptide wie Antikörper oder Fragmente davon, Enzyme, strukturelle Proteine usw.

"Operativ gebunden", wie hier verwendet, bedeutet, dass das Nukleinsäuremolekül kodierend für ein heterologes oder homologes Polypeptid auf eine Weise an den Promoter gebunden ist, die die Expression der Nukleotidsequenz in einer erfindungsgemäßen Hefezelle ermöglicht. Der Promoter ist somit an eine kodierende Nukleinsäuresequenz operativ gebunden, wenn dieser die Transkription der kodierenden Sequenz beeinflusst.

Gemäß einer weiteren bevorzugten Ausführungsform der vorliegenden Erfindung umfasst das heterologe oder homologe Polypeptid ein Signalpeptid, insbesondere ein Sekretionssignalpeptid.

Um ein rekombinant hergestelltes homologes oder heterologes Polypeptid aus der Hefezelle zu schleusen, umfasst das vom Nukleinsäuremolekül kodierte Polypeptid ein Signalpeptid.

"Signalpeptid", wie hier verwendet, bezieht sich auf ein am C- oder N-Terminus des Polypeptids gebundenes Peptid, das die Sezernierung des Polypeptids steuert. Die in der vorliegenden Erfindung verwendete Signalsequenz kann ein Polynukleotid sein, das eine Aminosäuresequenz kodiert, die den Transport eines Proteins durch die Membran des endoplasmatischen Retikulums (ER) initiiert. Die Nukleinsäuresequenz dieser Signalsequenzen kann der natürlichen Sequenz der ursprünglichen Wirtszelle entsprechen oder Codon optimiert sein. Die nicht einschränkenden Beispiele der Signalsequenz sind MF-alpha ("Mating-Faktor-Alpha"-Signalsequenz), die Signalsequenz der des CBH2 Proteins aus *Trichoderma reesei,* die Signalsequenz der Xylanase A aus *Thermomyces lanuginosus,* K1-Killertoxin-Signal, das Signalpeptid für die Invertase-Sekretion, die Signalsequenz des Killertoxins von *Kluyveromyces lactis,* die Signalsequenz des Killertoxins von *Pichia acaciae,* die Signalsequenz des Killertoxins von *Hanseniaspora uvarum* und von *Pichia (Hansenula) anomala* oder Varianten davon wie z.B. in Cereghino et al. (Gene 519(2013) :311-317) beschrieben. Die bevorzugte Signalsequenz der Erfindung ist MF-alpha ("Mating-Faktor-Alpha"-Signalsequenz).

Gemäß einer besonders bevorzugten Ausführungsform der vorliegenden Erfindung stammt der orthologe FMD Promoter von einer methylotrophen Hefezelle, die ausgewählt ist aus der Gruppe bestehend aus den Gattungen Hansenula (Ogataea), Candida, Komagataella und Pichia.

Gemäß einer weiteren bevorzugten Ausführungsform der vorliegenden Erfindung ist die methylotrophe Hefezelle ausgewählt aus der Gruppe bestehend aus *Hansenula polymorpha, Candida boidinii, Pichia methanolica, Komagataella pastoris, Komagataella phaffii, Komagataella populi, Komagataella pseudopastoris, Komagataella ulmi* und *Komagataella* sp. 11-1192.

Der orthologe FMD Promoter und das operativ daran gebundene Nukleinsäuremolekül kodierend für das heterologe oder homologe Polypeptid können im Genom, als extrachromosomales Nukleinsäurekonstrukt auf einem Plasmid mit autonom replizierender Sequenz (ARS) oder als Vektor/Expressionskassette ins Genom integriert vorliegen.

Der orthologe FMD Promoter und das operativ daran gebundene Nukleinsäuremolekül können extrachromosomal oder im Genom der erfindungsgemäßen Hefezelle integriert vorliegen.

Erfindungsgemäß umfasst der orthologe Promoter die Nukleinsäuresequenz SEQ ID Nr. 1.
SEQ ID Nr. 1:
SEQ ID Nr. 2:

Ein weiterer Aspekt der vorliegenden Erfindung betrifft ein Verfahren zur Herstellung eines heterologen Polypeptids umfassend den Schritt des Kultivierens einer Hefezelle gemäß der vorliegenden Erfindung.

Die erfindungsgemäße Hefezelle umfassend einen orthologen FMD Promoter eignet sich insbesondere zur Überexpression von homologen oder heterologen Polypeptiden. Aufgrund der hervorragenden Eigenschaften ist es mit der erfindungsgemäßen Hefezelle möglich ein Polypeptid bzw. Protein sowohl unter dereprimierenden als auch unter Methanol-induzierten oder geeigneten alternativen induzierenden Bedingungen zu exprimieren und gegebenenfalls aus der Zelle zu sekretieren.

Gemäß einer bevorzugten Ausführungsform der vorliegenden Erfindung wird während der Kultivierung die Expression des heterologen Polypeptids unter dereprimierenden Bedingungen induziert oder dessen Expressionsrate erhöht.

Promotor Derepression kann durch eine reduzierte Fütterungsrate mit einer reprimierenden Kohlenstoffquelle (C-Quelle: z.B. Glukose, Glyzerin) oder durch Verwendung einer nicht reprimierenden C-Quelle (z.B. Sorbitol) erreicht werden. Die reprimierende C-Quelle kann ihre Eigenschaften durch direkte Repression erreichen oder durch reprimierende Eigenschaften von Metaboliten der C-Quelle. Die Fütterungsrate mit reprimierenden C-Quellen kann im Extremfall gegen Null gehen. Auch zusätzliche Induktionseffekte durch andere Verbindungen wie z.B. durch Fettsäuren, Formaldehyd oder Ameisensäure sind möglich.

Um die Proteinausbeute während des Kultivierens bzw. während dessen Expression zu erhöhen, wird während der Kultivierung unter dereprimierenden Bedingungen vorzugsweise Methanol zugesetzt.

Die allgemeinen Kultivierungsbedingungen wie Temperatur, Medium usw. sind dem Fachmann hinreichend bekannt (siehe z.B. Krainer FW et al. Microbial Cell Factories 11(2012):22)

Die vorliegende Erfindung wird anhand der folgenden Figuren und Beispiele näher definiert, ohne jedoch auf diese beschränkt zu sein.

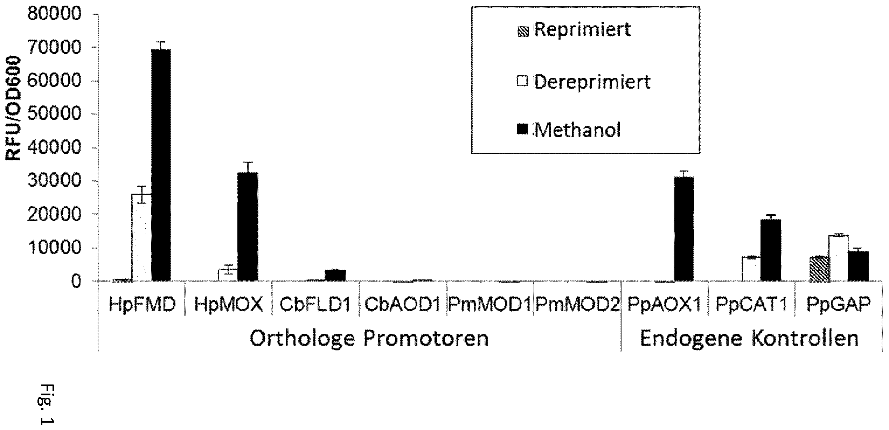

Fig. 1 zeigt die Fluoreszenzintensitäten eines grün fluoreszierenden Reporterproteins (eine verbesserte Variante des grünen fluoreszierenden Proteins (GFP)) bei Kultivierung von Hefezellen der Gattung Komagataella, bei denen eine für das grün fluoreszierende Protein kodierende Nukleinsäure operativ an orthologe und endogene Promotoren gebunden ist.. Die orthologen Promotoren (und endogene Promotoren aus *P. pastoris* als Referenz) wurden operativ an das GFP Reporter Gen gebunden und als Vektoren in *P. pastoris* transformiert. Die Stämme wurden in Mikrotiterplatten mit 96 Vertiefungen ('deep well plate' (DWP)) auf minimal Medium (BMD1) für 60 h kultiviert und anschließend mit Methanol induziert. Die Fluoreszenz des Reporterproteins und OD 600 (als Maß für Biomasse) wurde unter Glukose reprimierten Bedingungen (16 h), dereprimierten Bedingungen (60h) und verschiedenen Zeitpunkten nach Methanolinduktion gemessen. Die Fluoreszenzmessungen wurden in Bezug auf die OD600-Werte normalisiert. Mittelwerte und Standardabweichungen von jeweils vier Transformanten sind gezeigt.

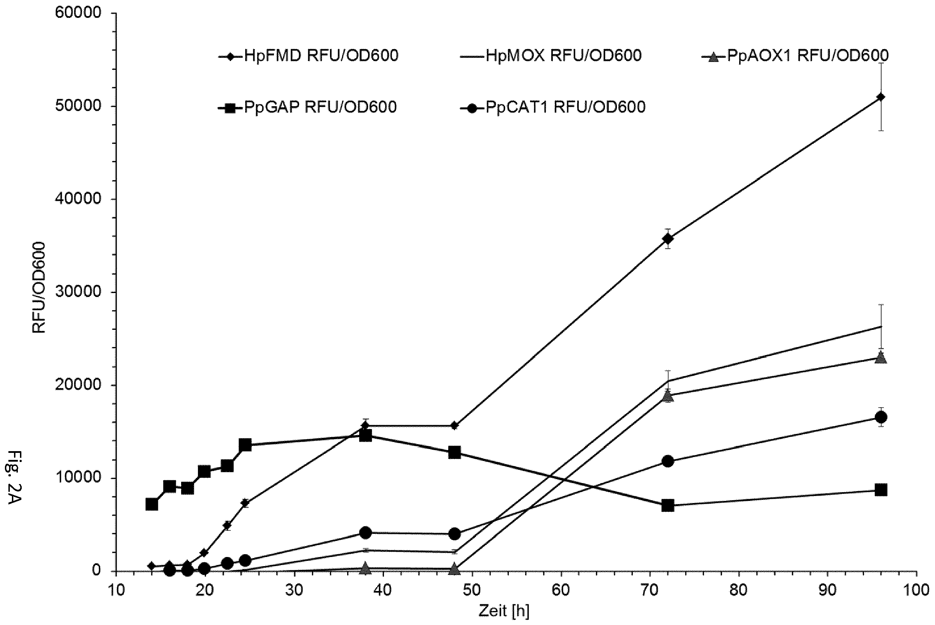

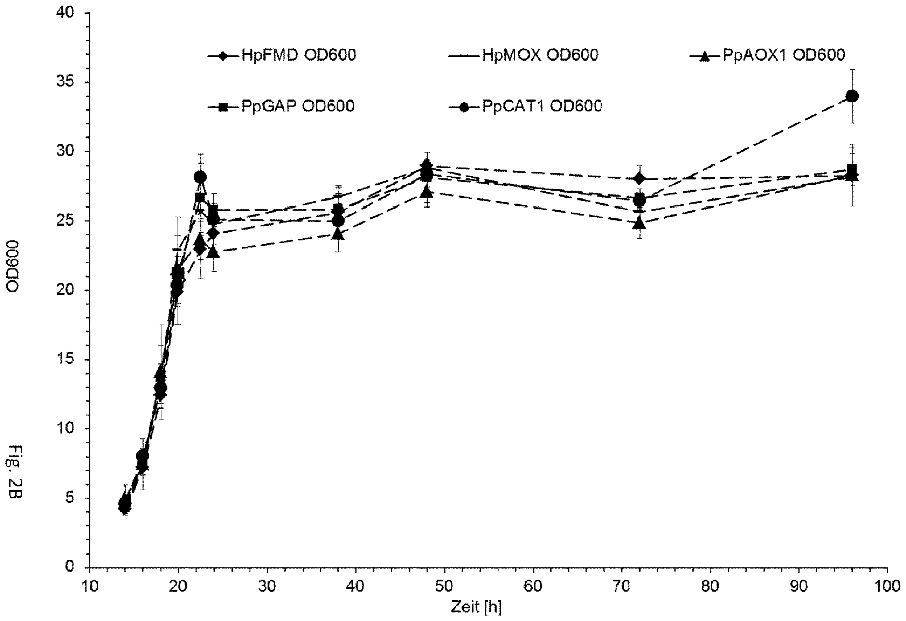

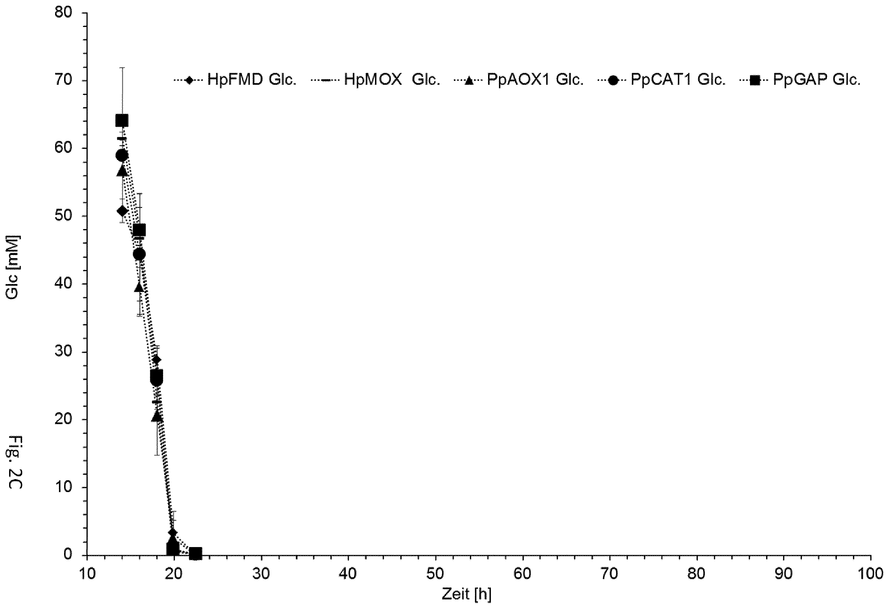

Fig. 2 zeigt den zeitlichen Verlauf von Messungen der Proteinexpression. Ausgewählte Stämme aus Fig.1 wurden in Schüttelkolben kultiviert. Die Proteinfluoreszenz (Fig. 2A; Verhältnis RFU/=D600; "RFU", "relative fluorescence unit", "relative Fluoreszenzeinheit"), die OD600 (Fig. 2B) und die Glukosemenge (Fig. 2C) wurde über die Zeit gemsssen. Die Glukosekonzentration am Beginn der Messungen betrug 55,5 mM (10 g/l). Mittelwerte (MV) und Standardabweichungen von jeweils drei Transformanten sind gezeigt.

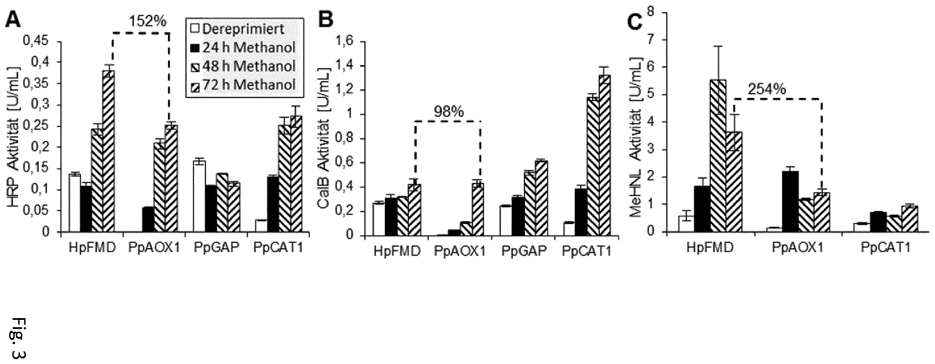

Fig. 3A bis 3C zeigen, dass der orthologe HpFMD Promoter auch in der Lage ist, die Expression anderer Reporterproteine wie Meerrettichperoxidase (HRP) (Fig. 3A), Lipase B aus Candida Antarctica (CalB) (Fig. 3B) und einer Hydroxynitrillyase aus Manihot esculenta (MeHNL) (Fig. 3C) hochzuregulieren. Die Stämme wurden in DWPs in Minimalmedium bis zum Glukoseverbrauch ("Depletion") nach 60 h kultiviert und anschließend zusätzlich mit Methanol induziert. HRP und CalB Enzymaktivitäten wurden im Kulturüberstand gemessen. Die Aktivität von MeHNL wurde mit aufgeschlossenen Zellen gemessen. Mittelwerte und Standardabweichungen von jeweils vier Transformanten sind gezeigt.

### BEISPIEL:

### Materialien und Methoden

### Klonierung der Promotoren

Die orthologen Promotoren wurden mittels PCR amplifiziert und vor ein GFP Reporter Gen kloniert. Dazu wurde das Reporter Plasmid pPpT4mutZeoMlyI-intARG4-eGFP-BmrIstuffer (Vogl T et al. ACS Synth Biol. 2015, DOI: 10.1021/acssynbio.5b00199; published on 22 November 2015) verwendet.

Dieses Plasmid basiert auf dem pPpT4 Vektor der von Näätsaari L et al. (PLoS One 7(2012):e39720) beschrieben wurde. Die Promotoren wurden nahtlos (d.h. ohne Restriktionsenzymschnittstellen oder Linker-Sequenzen zwischen Promoter und Start Codon) kloniert um den natürlichen Kontext zu erhalten. Primer wurden anhand von Literaturreferenzen designt (HpFMD Promotoren (Song H, et al. Biotechnol Lett 25(2003):1999-2006; Ledeboer AM, et al. Nucleic Acids Res 13(1985):3063-3082), CbAOD1 Promotor (Yurimoto H, et al. Biochim Biophys Acta 1493(2000):56-63), CbFLD1 Promotor (Lee B, et al. Microbiology 148(2000):2697-704), Pm MODI und MOD2 Promotoren (Raymond CK, et al. Yeast 14(1998):11-23; Nakagawa T, et al. J Biosci Bioeng 91(2001):225-7; Nakagawa T, et al. Yeast 23(2006):15-22). Die verwendeten Primersequenzen sind in Tabelle A angegeben:

**Tabelle A: Primer zur Amplifizierung der orthologen Promotoren**

| **Name** | **Sequenz** | **SEQ ID Nr.** |
|---|---|---|
| HpFMDfwd | AATGTATCTAAACGCAAACTCCGAGCTG | 3 |
| HpFMDrev | GATTTGATTGATGAAGGCAGAGAGCGCAAG | 4 |
| HpMOXfwd | TCGACGCGGAGAACGATCTCCTCGAGCT | 5 |
| HpMOXrev | | 6 |
| PmMOD1fwd | CGAGATGGTACATACTTAAAAGCTGCCATATTGAG | 7 |
| PmMOD1rev | | 8 |
| PmMOD2fwd | GGATCCACTACAGTTTACCAATTGATTACGCCAATAG | 9 |
| PmMOD2rev | | 10 |
| CbAODlfwd | | 11 |
| CbAOD1rev | | 12 |
| CbFLD1fwd | GGATCCCTTCAACAGCGGAGTCTCAAAC | 13 |
| CbFLD1rev | | 14 |

Genomische DNA der Stämme Hp (*Hansenula polymorpha*) DSM 70277, Cb (*Candida boidinii*) DSM 70026 and Pm (*Pichia methanolica*) DSM 2147 wurde isoliert und als Template für die PCR Reaktionen verwendet. Die PCR Produkte wurden durch TA Klonierungen ("TA cloning") in den Vektor pPpT4mutZeoMlyI-intARG4-eGFP-BmrIstuffer kloniert (siehe auch US 2015/0011407 und Vogl T et al. (ACS Synth Biol. 2015, DOI: 10.1021/acssynbio.5b00199; published on 22 November 2015)). Die Kontrollvektoren zu den P. *pastoris* endogenen Promotoren AOX1, CAT1 und GAP sind der US 2015/0011407 entnommen.

Die alternativen Reporter-Vektoren, die HRP (Isoenzym A2A; Näätsaari L, et al. BMC Genomics 15(2014):227), CalB und MeHNL downstream der entsprechenden Promotoren enthalten, wurden der US 2015/0011407 entnommen oder erstellt, indem das eGFP Reportergen aus den oben erwähnten eGFP Vektoren herausgeschnitten wurde (Restriktionsenzyme NheI und NotI) und nahtlos die PCR Produkte von HRP, CalB und MeHNL durch Rekombinationsklonierung ("recombination cloning") eingebaut wurden. Für die PCR Amplifikationen wurden die in Tabelle B angegebenen Primer verwendet

**Tabelle B: Primer für die Klonierung von Promotoren oberhalb ("upstream") verschiedener Reportergene**

| **Primer** | **Sequenz** | **SEQ ID Nr.** |
|---|---|---|
| pHpFMD-MFalpha-Gib | | 15 |
| AOX1TT-NotI-CalB | | 16 |
| AOX1TT-NotI-HRPA2A | | 17 |
| seq-pHpHMD-149..126fwd | actggtgtccgccaataagaggag | 18 |
| pHpFMD-MeHNL | | 19 |
| AOX1TT-NotI-MeHNL | | 20 |
| pCAT1-MeHNL-Gib | | 21 |

Hierzu wurden die in Literatur erwähnten HRP und CalB Vektoren als PCR Template verwendet (US 2015/0011407) und Vogl T. et al. (ACS Synth Biol. 2015, DOI: 10.1021/acssynbio.5b00199; published on 22 November 2015). Die MeHNL Sequenz wurde für *P. pastoris* Codon optimiert und als synthetisches doppelsträngiges DNA Fragment mit Überhängen zum AOX1 Promoter und Terminator designt (siehe Tabelle B). Dieses Fragment wurde als Template für PCRs verwendet. Die folgende Sequenz wurde verwendet:

Die Protein kodierende Sequenz ist hier groß, Start und Stopp Codon fett geschrieben, Überhänge zu dem Vektor für Rekombinationsklonierung sind klein geschrieben, EcoRI und NotI Schnittstellen, die typischerweise für Klonierungen in die pPpT4 Vektor Familie verwendet werden sind unterstrichen.

Für die PCR Amplifizierung der HRP und CalB Gene konnte der selbe Vorwärts-Primer (pHpFMD-MFalpha-Gib) verwendet werden, da beide Gene mit einer MFalpha Signal Sequenz fusioniert sind. Die in die Vektoren klonierten Gene wurden durch Primer, die in den AOX1 Terminator und die respektiven Promotoren binden, sequenziert (seq-pHpHMD-149..126fwd für den HpFMD Promoter).

### Stämme, Materialen, Fluoreszenzmessungen und Enzymassays

Enzymatische HRP und CalB Aktivität wurden mit den Substraten 2,2'-azino-bis(3-ethylbenzthiazoline-6-sulphonic acid)diammoniumsalt(ABTS) und p-nitrophenyl butyrate (p-NPB) bestimmt nach den Protokollen in Krainer FW (Microb Cell Fact11 (2012) :22) .

Für die Transformationen aller Promotervergleiche mit GFP wurde der CBS7435 Wildtyp Stamm verwendet. HRP und CalB Plasmide wurden in den mutS Stamm transformiert (Näätsaari et al., PLoS One 2012;7:e39720), da dieser höhere Proteinexpression zeigt (Krainer FW, et al. Microb Cell Fact 11(2012):22). Für MeHNL Aktivitätsmessungen wurden die Zellen durch Y-PER (Thermo Fisher Scientific, Y-PER™ Yeast Protein Extraction Reagent) Aufschluss nach Herstellerangaben lysiert und die Aktivität mit einem "Mandelonitrile Cyanogenese Assay" gemessen wie in Wiedner R et al. Comput Struct Biotechnol J10(2014):58-62) beschrieben (finale Mandelonitril Konzentration 15 mM).

### Resultate

Sechs heterologe Promotoren von den HpFMD, HpMOX, CbFLD1, CbAOD1, PmMOD1 und PmMOD2 Genen wurden in *P. pastoris* getestet. Die Promotoren wurden mit dem Methanol induzierbaren AOX1 Promoter, dem konstitutiven GAP Promoter und dem dereprimierten/Methanol induzierbaren CAT1 Promoter in P. pastoris verglichen, nämlich. Die orthologen Promotoren wurden von genomischer DNA PCR amplifiziert und in Vektor mit GFP als Reportergen kloniert. Die folgenden Promoter Sequenzen wurden verwendet:
HpFMD (SEQ ID Nr. 1):
HpMOX (SEQ ID Nr. 2):
(SEQ ID Nr. 23):
CbAOD1 (SEQ ID Nr. 24):
PmMOD1 (SEQ ID Nr. 25):
PmMOD2 (SEQ ID Nr. 26):

P. pastoris Transformanten, die Plasmide mit den Promotoren CbAOD1, PmMOD1 und PmMOD2 enthielten zeigte keine Reporter Protein Fluoreszenz (Fig. 1). Der Promoter zeigte Repression auf Glukose und schwache Induktion durch Methanol von etwa 10% des PpAOX1 Promoters. Überraschenderweise behielten beide getesteten *H. polymorpha* Promotoren ihr natürliches Regulationsprofil aus *H. polymorpha* und zeigten auch in Pichia pastoris Repression, Derepression und Methanol Induktion (Fig. 1 und 2). Der HpFMD Promoter übertraf erstaunlicherweise unter dereprimierten Bedingungen den konsitutiven PpGAP Promoter und erreichte sogar ohne weitere C-Quellen Fütterung zur Energieversorgung etwa 75% des Methanol induzierten PpAOX1 Promoters. Die dereprimierte Expression des HpFMD Promoters übertraf die Repoter Protein Fluoreszenz des stärksten endogenen MUT Promoters aus P. pastoris (PpCAT1) zirka 3,5 fach. Nach Methanolinduktion übertraf der HpFMD Promoter sogar den PpAOX1 Promoter etwa zweifach. Diese Resultate im kleinen Maßstab (Fig. 1) wurden durch Experimente in Schüttelflaschen bestätigt (Fig. 2), wobei durch Glukose Messungen auch klar das dereprimierte Regulationsprofil gezeigt wurde. Eine weitere Verstärkung der technischen Vorteile des HpFMD Promotors ist durch eine optimierte Fütterungsrate im Bioreaktor erreichbar.

Um zu untersuchen, ob die unerwartet starke Expression des HpFMD Promoters auch für andere Proteine als GFP reproduziert werden können, wurde der HpFMD Promtoer vor die kodierenden Sequenzen von weiteren Proteinen kloniert: Den sekretierten Proteinen Meerrettichperoxidase (HRP) und Candida antarctica Lipase B (CalB) und der intrazellulären Hydroxynitrillyase aus Manihot esculenta (MeHNL) (Fig. 3A bis 3C).

In Bezug auf die finalen Ausbeuten an aktivem Protein im Kulturüberstand im Schüttelkolbenexperiment kam die dereprimierte Expression aller Proteine durch den HpFMD Promoter der konstitutiven Expression durch den GAP Promoter gleich und übertraf klar die dereprimierte Expression durch den CAT1 Promoter. Methanol induzierte Enzymaktivitäten des HpFMD Promoters übertrafen die AOX1 Promoteraktivität um das 2,5-fache.

Die starke Expression des HpFMD Promoters war auch bei vier verschiedenen sekretierten als auch intrazellulären Reporterproteinen (eGFP, HRP, CalB, MeHNL) beobachtbar. Der orthologe HpFMD Promoter übertraf sogar endogene Promotoren in P. pastoris.

Interessanterweise zeigen die orthologen Promotoren keine bzw. sehr niedrige Sequenzidentitäten zu Promotoren in Pichia. Eine BLAST Suche des HpFMD Promoters ergab keine signifikanten Treffer im Pichia pastoris Genom, auch ein direktes Alignment des HpFMD Promoters zu dem PpFDH1 Promoter ergab keine signifikanten Ähnlichkeiten (BLASTN 2.2.32+, Blast 2 sequences, setting for ,Somewhat similar sequences (blastn)', Molecule type: nucleic acid).

Solche niedrige Sequnzidentität ist eine wünschenswerte Eigenschaft der Promotoren, da diese fremden Sequenzen nicht mit identen Sequnenzen in dem Genom von Pichia rekombinieren können und so nicht z.B. durch homologe Rekombinationsereignisse mit bereits im genom vorhandenen ähnlichen Sequenzen verloren gehen können.

Überraschenderweise können orthologe Promotoren höchst nützliche Werkzeuge für Proteinexpression darstellen, wie durch bis zu 2,5-fach höhere Aktivitäten durch den HpFMD Promoter gezeigt. Unerwarteter Weise behielt der HpFMD Promoter auch in P. pastoris sein dereprimertes Regulationsprofil aus H. polymorpha und stellt damit den stärksten dereprimierten Promoter in P. pastoris dar. Dadurch können effiziente Produktionsprozesse frei von toxischem und hoch entzündlichem Methanol ermöglicht werden.

## Patentansprüche

1. Pichia pastoris Zelle umfassend einen orthologen durch Derepression induzierbaren Promoter einer methylotrophen Hefezelle oder eine durch Derepression induzierbare Variante davon, **dadurch gekennzeichnet, dass** der orthologe Promoter ein orthologer Formiatdehydrogenase (FMD) Promoter einer methylotrophen Hefezelle und die Variante mindestens 90% ident mit dem Promoter ist, wobei der orthologe Promoter eine Nukleinsäuresequenz SEQ ID Nr. 1 aufweist und operativ an ein Nukleinsäuremolekül kodierend für ein heterologes oder homologes Polypeptid gebunden ist.

2. Pichia pastoris Zelle nach Anspruch 1, **dadurch gekennzeichnet, dass** der orthologe Promoter mit Methanol induzierbar ist.

3. Pichia pastoris Zelle nach Anspruch 1 oder 2, **dadurch gekennzeichnet, dass** das heterologe oder homologe Polypeptid ein Signalpeptid, insbesondere ein Sekretionssignalpeptid, umfasst.

4. Pichia pastoris Zelle nach einem der Ansprüche 1 bis 3, **dadurch gekennzeichnet, dass** der orthologe Promoter von einer methylotrophen Hefezelle stammt, die ausgewählt ist aus der Gruppe bestehend aus den Gattungen Hansenula, Candida, Komagataella und Pichia.

5. Pichia pastoris Zelle nach einem der Ansprüche 1 bis 4, **dadurch gekennzeichnet, dass** die methylotrophe Hefezelle ausgewählt ist aus der Gruppe bestehend aus Hansenula polymorpha, Candida boidinii, Pichia methanolica, Komagataella pastoris, Komagataella phaffii, Komagataella populi, Komagataella pseudopastoris, Komagataella ulmi und Komagataella sp. 11-1192.

6. Pichia pastoris Zelle nach einem der Ansprüche 1 bis 5, **dadurch gekennzeichnet, dass** der orthologe Promoter und gegebenenfalls das operativ daran gebundene Nukleinsäuremolekül kodierend für das heterologe oder homologe Polypeptid im Genom oder als extrachromosomales Nukleinsäurekonstrukt vorliegt.

7. Verfahren zur Herstellung eines heterologen Polypeptids umfassend den Schritt des Kultivierens einer Pichia pastoris Zelle gemäß einem der Ansprüche 1 bis 6.

8. Verfahren nach Anspruch 7, **dadurch gekennzeichnet, dass** während der Kultivierung die Expression des heterologen Polypeptids durch dereprimierende Bedingungen induziert oder dessen Expressionsrate erhöht wird.

9. Verfahren nach Anspruch 7, **dadurch gekennzeichnet, dass** während der Kultivierung unter dereprimierenden Bedingungen Methanol oder ein alternativer Induktor zugesetzt wird.

## Claims

1. A pichia pastoris cell comprising an orthologous promoter of a methylotrophic yeast cell inducible by derepression or a variant thereof inducible by derepression, **characterized in that** the orthologous promoter is an orthologous formate dehydrogenase (FMD) promoter of a methylotrophic yeast cell and the variant is at least 90% identical to the promoter, wherein the orthologous promoter comprises a nucleic acid sequence SEQ ID No. 1 and is operably linked to a nucleic acid molecule coding for a heterologous or homologous polypeptide.

2. The pichia pastoris cell according to claim 1, **characterized in that** the orthologous promoter is inducible with methanol.

3. The pichia pastoris cell according to claim 1 or 2, **characterized in that** the heterologous or homologous polypeptide comprises a signal peptide, in particular a secretion signal peptide.

4. The pichia pastoris cell according to any of claims 1 to 3, **characterized in that** the orthologous promoter from a methylotrophic yeast cell selected from the group consisting of the genera Hansenula, Candida, Komagataella and Pichia.

5. The pichia pastoris cell according to any of claims 1 to 4, **characterized in that** the methylotrophic yeast cell is selected from the group consisting of Hansenula polymorpha, Candida boidinii, Pichia methanolica, Komagataella pastoris, Komagataella phaffii, Komagataella populi, Komagataella pseudopastoris, Komagataella ulmi and Komagataella sp. 11-1192.

6. The pichia pastoris cell according to any of claims 1 to 5, **characterized in that** the orthologous promoter and optionally the nucleic acid molecule coding for the heterologous or homologous polypeptide operably linked to the promoter are present in the genome or as an extrachromosomal nucleic acid construct.

7. A method for producing a heterologous polypeptide comprising the step of culturing a pichia pastoris cell according to any of claims 1 to 6.

8. The method according to claim 7, **characterized in that** during the culturing, the expression of the heterologous polypeptide is induced or its expression rate is increased by derepressing conditions.

9. The method according to claim 7, **characterized in that** during the culturing under derepressing conditions, methanol or an alternative inductor is added.

## Revendications

1. Cellule de Pichia pastoris comportant un promoteur orthologue, inductible par dérépression, d'une cellule de levure méthylotrophe ou un variant de celui-ci inductible par dérépression, **caractérisée en ce que** le promoteur orthologue est un promoteur orthologue de formiate déshydrogénase (FMD) d'une cellule de levure métylotrophe et le variant est au moins à 90 % identique au promoteur, dans lequel le promoteur orthologue présente une séquence d'acide nucléique SEQ N° 1 et est lié de manière opérationnelle à une molécule d'acide nucléique codant pour un polypeptide hétérologue ou homologue.

2. Cellule de Pichia pastoris selon la revendication 1, **caractérisée en ce que** le promoteur orthologue est inductible par le méthanol.

3. Cellule de Pichia pastoris selon la revendication 1 ou 2, **caractérisée en ce que** le polypeptide hétérologue ou homologue comporte un peptide signal, en particulier un peptide signal de sécrétion.

4. Cellule de Pichia pastoris selon l'une quelconque des revendications 1 à 3, **caractérisée en ce que** le promoteur orthologue provient d'une cellule de levure méthylotrophe, qui est choisie dans le groupe constitué par les genres Hansenula, Candida, Komagataella et Pichia.

5. Cellule de Pichia pastoris selon l'une quelconque des revendications 1 à 4, **caractérisée en ce que** la cellule de levure méthylotrophe est choisie dans le groupe constitué par Hansenula polymorpha, Candida boidinii, Pichia methanolica, Komagataella pastoris, Komagataella phaffii, Komagataella populi, Komagatella pseudopastoris, Komagataella ulmi et Komagataella sp. 11-1192.

6. Cellule de Pichia pastoris selon l'une quelconque des revendications 1 à 5, **caractérisée en ce que** le promoteur orthologue et le cas échéant la molécule d'acide nucléique liée de manière opérationnelle à celui-ci, codant pour le polypeptide hétérologue ou homologue, est présent(e) dans le génome ou sous la forme d'un produit de synthèse d'acide nucléique extrachromosomique.

7. Procédé d'obtention d'un polypeptide hétérologue comportant l'étape de culture d'une cellule de Pichia pastoris selon l'une quelconque des revendications 1 à 6.

8. Procédé selon la revendication 7, **caractérisé en ce que**, pendant la culture, l'expression du polypeptide hétérologue est induite par des conditions de dérépression ou augmentée par la vitesse d'expression de celui-ci.

9. Procédé selon la revendication 7, **caractérisé en ce que** pendant la culture dans des conditions de dérépression, du méthanol ou un autre inducteur est ajouté.


### SEQUENCE LISTING

<110> Technische Universität Graz
<120> Hefezelle
<130> 47851
<160> 26
<170> PatentIn version 3.5
<210> 1
   <211> 623
   <212> DNA
   <213> Hansenula polymorpha
<400> 1
   aatgtatcta aacgcaaact ccgagctgga aaaatgttac cggcgatgcg cggacaattt        60
   agaggcggcg atcaagaaac acctgctggg cgagcagtct ggagcacagt cttcgatggg       120
   cccgagatcc caccgcgttc ctgggtaccg ggacgtgagg cagcgcgaca tccatcaaat       180
   ataccaggcg ccaaccgagt ctctcggaaa acagcttctg gatatcttcc gctggcggcg       240
   caacgacgaa taatagtccc tggaggtgac ggaatatata tgtgtggagg gtaaatctga       300
   cagggtgtag caaaggtaat attttcctaa aacatgcaat cggctgcccc gcaacgggaa       360
   aaagaatgac tttggcactc ttcaccagag tggggtgtcc cgctcgtgtg tgcaaatagg       420
   ctcccactgg tcaccccgga ttttgcagaa aaacagcaag ttccggggtg tctcactggt       480
   gtccgccaat aagaggagcc ggcaggcacg gagtctacat caagctgtct ccgatacact       540
   cgactaccat ccgggtctct cagagagggg aatggcacta taaataccgc ctccttgcgc       600
   tctctgcctt catcaatcaa atc                                               623
<210> 2
   <211> 1510
   <212> DNA
   <213> Hansenula polymorpha
<400> 2
   cgacgcggag aacgatctcc tcgagctgct cgcggatcag cttgtggccc ggtaatggaa        60
   ccaggccgac ggcacgctcc ttgcggacca cggtggctgg cgagcccagt ttgtgaacga       120
   ggtcgtttag aacgtcctgc gcaaagtcca gtgtcagatg aatgtcctcc tcggaccaat       180
   tcagcatgtt ctcgagcagc catctgtctt tggagtagaa gcgtaatctc tgctcctcgt       240
   tactgtaccg gaagaggtag tttgcctcgc cgcccataat gaacaggttc tctttctggt       300
   ggcctgtgag cagcggggac gtctggacgg cgtcgatgag gcccttgagg cgctcgtagt       360
   acttgttcgc gtcgctgtag ccggccgcgg tgacgatacc cacatagagg tccttggcca       420
   ttagtttgat gaggtggggc aggatgggcg actcggcatc gaaatttttg ccgtcgtcgt       480
   acagtgtgat gtcaccatcg aatgtaatga gctgcagctt gcgatctcgg atggttttgg      540
   aatggaagaa ccgcgacatc tccaacagct gggccgtgtt gagaatgagc cggacgtcgt      600
   tgaacgaggg ggccacaagc cggcgtttgc tgatggcgcg gcgctcgtcc tcgatgtaga      660
   aggccttttc cagaggcagt ctcgtgaaga agctgccaac gctcggaacc agctgcacga      720
   gccgagacaa ttcgggggtg ccggctttgg tcatttcaat gttgtcgtcg atgaggagtt      780
   cgaggtcgtg gaagatttcc gcgtagcggc gttttgcctc agagtttacc atgaggtcgt      840
   ccactgcaga gatgccgttg ctcttcaccg cgtacaggac gaacggcgtg gccagcaggc      900
   ccttgatcca ttctatgagg ccatctcgac ggtgttcctt gagtgcgtac tccactctgt      960
   agcgactgga catctcgaga ctgggcttgc tgtgctggat gcaccaatta attgttgccg     1020
   catgcatcct tgcaccgcaa gtttttaaaa cccactcgct ttagccgtcg cgtaaaactt     1080
   gtgaatctgg caactgaggg ggttctgcag ccgcaaccga acttttcgct tcgaggacgc     1140
   agctggatgg tgtcatgtga ggctctgttt gctggcgtag cctacaacgt gaccttgcct     1200
   aaccggacgg cgctacccac tgctgtctgt gcctgctacc agaaaatcac cagagcagca     1260
   gagggccgat gtggcaactg gtggggtgtc ggacaggctg tttctccaca gtgcaaatgc     1320
   gggtgaaccg gccagaaagt aaattcttat gctaccgtgc agtgactccg acatccccag     1380
   tttttgccct acttgatcac agatggggtc agcgctgccg ctaagtgtac ccaaccgtcc     1440
   ccacacggtc catctataaa tactgctgcc agtgcacggt ggtgacatca atctaaagta     1500
   caaaaacaaa                                                            1510
<210> 3
   <211> 28
   <212> DNA
   <213> Artificial Sequence
<220>
   <223> Primer
<400> 3
   aatgtatcta aacgcaaact ccgagctg 28
<210> 4
   <211> 30
   <212> DNA
   <213> Artificial Sequence
<220>
   <223> Primer
<400> 4
   gatttgattg atgaaggcag agagcgcaag 30
<210> 5
   <211> 28
   <212> DNA
   <213> Artificial Sequence
<220>
   <223> Primer
<400> 5
   tcgacgcgga gaacgatctc ctcgagct 28
<210> 6
   <211> 45
   <212> DNA
   <213> Artificial Sequence
<220>
   <223> Primer
<400> 6
   tttgtttttg tactttagat tgatgtcacc accgtgcact ggcag 45
<210> 7
   <211> 35
   <212> DNA
   <213> Artificial Sequence
<220>
   <223> Primer
<400> 7
   cgagatggta catacttaaa agctgccata ttgag 35
<210> 8
   <211> 56
   <212> DNA
   <213> Artificial Sequence
<220>
   <223> Primer
<400> 8
   tttgagaaat taatagtaag attttttttt cgtaaaagtt ttgattgagt taattc 56
<210> 9
   <211> 37
   <212> DNA
   <213> Artificial Sequence
<220>
   <223> Primer
<400> 9
   ggatccacta cagtttacca attgattacg ccaatag 37
<210> 10
   <211> 60
   <212> DNA
   <213> Artificial Sequence
<220>
   <223> Primer
<400> 10
   tttgaatttt agttttagat agataaatat aattttcaat cctgttataa aatagtatat 60
<210> 11
   <211> 55
   <212> DNA
   <213> Artificial Sequence
<220>
   <223> Primer
<400> 11
   ggagtatacg taaatatata attatatata atcatatata tgaatacaat gaaag 55
<210> 12
   <211> 58
   <212> DNA
   <213> Artificial Sequence
<220>
   <223> Primer
<400> 12
   tattgaaaaa taattttgtt tttttttttt tgttttttta aaagttcgtt aaaattcg 58
<210> 13
   <211> 28
   <212> DNA
   <213> Artificial Sequence
<220>
   <223> Primer
<400> 13
   ggatcccttc aacagcggag tctcaaac 28
<210> 14
   <211> 58
   <212> DNA
   <213> Artificial Sequence
<220>
   <223> Primer
<400> 14
   ttttgtggaa taaaaaatag ataaatatga tttagtgtag ttgattcaat caattgac 58
<210> 15
   <211> 60
   <212> DNA
   <213> Artificial Sequence
<220>
   <223> Primer
<400> 15
   cttgcgctct ctgccttcat caatcaaatc atgagattcc catctatttt caccgctgtc 60
<210> 16
   <211> 60
   <212> DNA
   <213> Artificial Sequence
<220>
   <223> Primer
<400> 16
   caaatggcat tctgacatcc tcttgagcgg ccgcttatgg ggtcacgata ccggaacaag 60
<210> 17
   <211> 66
   <212> DNA
   <213> Artificial Sequence
<220>
   <223> Primer
<400> 17
   caaatggcat tctgacatcc tcttgagcgg ccgcttagga tccgttaact ttcttgcaat 60
caagtc 66
<210> 18
   <211> 24
   <212> DNA
   <213> Artificial Sequence
<220>
   <223> Primer
<400> 18
   actggtgtcc gccaataaga ggag 24
<210> 19
   <211> 60
   <212> DNA
   <213> Artificial Sequence
<220>
   <223> Primer
<400> 19
   cttgcgctct ctgccttcat caatcaaatc atggttactg ctcacttcgt cttgattcac 60
<210> 20
   <211> 61
   <212> DNA
   <213> Artificial Sequence
<220>
   <223> Primer
<400> 20
   caaatggcat tctgacatcc tcttgagcgg ccgcttaagc gtaagcgtcg gcaacttcct 60
   g 61
<210> 21
   <211> 60
   <212> DNA
   <213> Artificial Sequence
<220>
   <223> Primer
<400> 21
   cacttgctct agtcaagact tacaattaaa atggttactg ctcacttcgt cttgattcac 60
<210> 22
   <211> 868
   <212> DNA
   <213> Artificial Sequence
<220>
   <223> Artificial Sequence
<400> 22
   cgacaacttg agaagatcaa aaaacaacta attattgaaa gaattccgaa acgatggtta 60
   ctgctcactt cgtcttgatt cacactatct gtcatggtgc ttggatctgg cacaagttga 120
   agccagcatt ggagagagct ggacataagg ttaccgctct tgatatggct gcatctggta 180
   ttgatcctcg tcaaatcgaa caaatcaatt cattcgacga gtactcagag ccactgctga 240
   ccttcttgga aaagttgcct caaggtgaaa aggtgatcat cgttggtgaa tcctgtgctg 300
   gattgaacat tgccattgca gctgatagat atgtcgataa gatcgctgct ggtgtcttcc 360
   acaactctct gttaccagat actgttcact ctccatctta cactgtcgag aagttgttag 420
   aatcattccc agattggaga gatactgaat actttacttt cactaacatc actggagaga 480
   ctatcaccac catgaaactt ggattcgttt tgttgagaga aaaccttttc accaagtgta 540
   ctgatggtga atacgaattg gccaagatgg ttatgagaaa gggttctttg tttcagaatg 600
   ttcttgcaca aagaccaaag ttcaccgaaa agggttacgg ttctatcaag aaggtctaca 660
   tctggactga tcaggacaag atcttcctgc cagacttcca aagatggcaa atcgcaaact 720
   acaaaccaga taaggtctac caagtccaag gtggtgatca caagttacaa ttgaccaaga 780
   ccgaagaggt cgctcacatc ttgcaggaag ttgccgacgc ttacgcttaa gcggccgctc 840
   aagaggatgt cagaatgcca tttgcctg 868
<210> 23
   <211> 572
   <212> DNA
   <213> Candida boidinii
<400> 23
   ggatcccttc aacagcggag tctcaagcag tggctattat cagtgtattt aattactgat      60
   gcattgtatt atagtgcata catagttaat aattactctc tgttatcatt gaaaattttg     120
   aaattctcac tctcacgcag tgcaaaactt tgcctaattg agtaagtgga acgcaatatt     180
   taggctacat attttggatt cccttaagta tgtaatcaaa gatcattcat actgccatct     240
   tataatattg gagtattatt atgttgctat actgttctac ctgtttattc tattgtatgc     300
   gtctaaatct ttccatcagt ttctatacta tctttcgttt gcaatgaaat attactccaa     360
   ttcgcttgtt tcaactcgct tgccttctct cttgccttct ttttttcttt tcattttatc     420
   gttgtttaaa cggtatataa atatgtaacg ttgtcgctta gttttgagaa atcacttttg     480
   ttgctctcaa ttctgttttg acatcttaag gttagtcaat tgattgaatc aactacacta     540
   aatcatattt atctattttt tattccacaa aa                                   572
<210> 24
   <211> 1652
   <212> DNA
   <213> Candida boidinii
<400> 24
   ggagtatacg taaatatata attatatata atcatatata tgaatacaat gcaatgaaag 60
   tgaatatgat aagattgaaa taataacaaa cagcgataaa tatatctcaa aatggagtta 120
   cacaacaaat aataataaaa tataaattat aaattataaa ttataaaaga ataaaaaata 180
   aaccccacta atttatttta ttaaaagata gattggtatc tttacttaat aacaattctg 240
   aaactttatt cacttaattt tatttaactt atttaattta tttttacccc agtttttcag 300
   tacaatgcag ctccgaaact ttatttggct gtgatttggc tgtgatttgg ctgtgatttg 360
   gcttggcttg gctggctgga attgtctcct gcaggaattg ctcggggtcc ggttctcccg 420
   ctggctggct atttggcggg ctggctattt ggcgggctgg ctggctggct gctctgccat 480
   ctgctgtggc caccccgcat ctctggatgc acgccgtgca gctggacgtg cgtctaccct 540
   gcagccgtgt gccttatttc ccaatctccc aatctctcaa tctgccagtc agccaaaaca 600
   ccggccaggc aggcaggcag gcaggcaggc aggcagtgaa gccttcccac gccccactcc 660
   gcataaacat ccccagcagt ttccccagca gtttccccag cttttcaatt taataaaata 720
   gcctgtttct gtttctgttt tatattatac aattttttat cctaataatt actctttcgg 780
   gaattaaata ataattatat catataccca tatcacattt tactatattt actatctata 840
   aataaattca tattataata ttaatttata ttcgcttaat taaaatgctc ttttccatca 900
   tcatcatcat catcatcatc acgagttttc ggttatcaat actcttttca ttaatttcta 960
   gaatttcatt atttattttt tattgactgg aaattttcaa tcaattttat ttatttttat 1020
   ttatttattt tcatattctt agatttaaac tttttagatg accgctattt tacttactta 1080
   cttactgttg ttttatatta tgataagaat taattttcat atttatgatg atgatgatgt 1140
   aaatttaacc tagtatacta ttttaaagtt atcactatct tttagtgctg gcatttttta 1200
   ttctattttc atatatgtat atacgtaaat taagtatcat cacgctgctt actgtacgtt 1260
   taaaatgtgg agatggaaat agagatgggg atgaagatga agatgatgag aattataaac 1320
   cattcattca ttaatcaatc aatataactt ataaaaaaat ttatatttaa atgaattaat 1380
   ttcctttatt ttaataatat cgttaattct tttaaattct attttatttt aattctttct 1440
   ttatcatagt tatcatataa caattatata acatagatac acaattatta tttcattatc 1500
   atattatttt ttaaaatatt gattattttt aaaataatat cttaattaat taatttttac 1560
   gaatatacaa attttaacga cttacttttt ttaacgaatt ttaacgaact tttaaaaaaa 1620
   caaaaaaaaa aaaacaaaat tatttttcaa ta 1652
<210> 25
   <211> 1157
   <212> DNA
   <213> Pichia methanolica
<400> 25
   cgagatggta catacttaaa agctgccata ttgaggaact tcaaagtttt atctgttttt     60
   agaattaaaa gacgattgtt gtaacaaaac gttgtgccta cataaactca aattaatgga    120
   aatagcctgt tttgaaaaat acaccttctt aagtactgac aaagttttgt taaatgacta    180
   tcgaacaagc catgaaatag cacatttctg ccagtcactt ttaacacttt cctgcttgct    240
   ggttgactct cctcatacaa acacccaaaa gggaaacttt cagtgtgggg acacttgaca    300
   tctcacatgc accccagatt aatttcccca gacgatgcgg agacaagaca aaacaaccct    360
   ttgtcctgct cttttctttc tcacaccgcg tgggtgtgtg cgcaggcagg caggcaggca    420
   gcgggctgcc tgccatctct aatcgctgct cctcccccct ggcttcaaat aacagcctgc    480
   tgctatctgt gaccagattg ggacaccccc ctcccctccg aatgatccat caccttttgt    540
   cgtactccga caatgatcct tccctgtcat cttctggcaa tcagctcctt caataattaa    600
   atcaaataag cataaatagt aaaatcgcat acaaacgtca tgaaaagttt tatctctatg    660
   gccaacggat agtctatctg cttaattcca tccactttgg gaaccgttct ctctttaccc    720
   cagattctca aagctaatat ctgccccttg tctattgtcc tttctccgtg tacaagcgga    780
   gcttttgcct cccatcctct tgctttgttt cggttatttt tttttctttt gaaactcttg    840
   gtcaaatcaa atcaaacaaa accaaacctt ctattccatc agatcaacct tgttcaacat    900
   tctataaatc gatataaata taaccttatc cctcccttgt tttttaccaa ttaatcaatc    960
   ttcaaatttc aaatattttc tacttgcttt attactcagt attaacattt gtttaaacca   1020
   actataactt ttaactggct ttagaagttt tatttaacat cagtttcaat ttacatcttt   1080
   atttattaac gaaatcttta cgaattaact caatcaaaac ttttacgaaa aaaaaatctt   1140
   actattaatt tctcaaa                                                  1157
<210> 26
   <211> 1662
   <212> DNA
   <213> Pichia methanolica
<400> 26
   ggatccacta cagtttacca attgattacg ccaatgtgtt tatttcacca agtaattaca      60
   aaactgagat ttggttatgt cattatgtat tttcggcaat ggctgtaatt taaactggat     120
   tagggttaat taacgtttag cctacgaaag cggctagctt ttatttctgc ttttgttttg     180
   agcccgtttc taattccaat ctttgcaatt tcgttccatc ttttaaaatt aagtgctctt     240
   ttctaatctg ataaagataa gccatcgtag agtaagtaaa acaaaataat gtactgtata     300
   ttaagcggaa aaacttggaa aagtcgtatg atgttgaagg agcaaagaat gactaatatt     360
   aggagattta agcaaacaat gttgagggga acaggacgat taacccctta tagaggaagc     420
   gtctttgatg ttcgaagggg gaggggtcaa aagcactgag cagtgctaat tagtaaccaa     480
   tttctgtaag caatgaaact tgttgctatt ggaaatacta ttaagtaata caaggtacag     540
   actaatgggg gtgagccggt agttcaggct atcttataga cagactattc cggattgtct     600
   aatcattggt gcacctggtt aataattatc agtcaactct tttacggtgc tgataggtct     660
   ttgcgaactt gcccttgtgg aatttggttg ttaatcaaac tgttctgtat ttcatgtcat     720
   actactattg atattattaa tgttacttac tcatctggcc atttaacagg tttgaagctt     780
   taatgctctt aactaacagc aatccatcac cgtcaacctt aacccccctg gtgcttgctg     840
   tctttatcct tcgtatcttt ttcatgttgc accgccctgt tccttatacg gttgttcccc     900
   cataggctaa cttctctgtt tccgaccatc tctgcaataa caaagaattc tatacgctta     960
   cactataatc atacaatgac tctacatgcc attttcactt tacttacttg ccatcggaag    1020
   atactgaatc agaaagccat agtaactaca taacttcaaa acacaccctt tttacagatt    1080
   agttacaatt ttgtcaatgt ttgtttgata acccaaggtg gaacgtttcc agttagacct    1140
   gtttaatcca actcacttta ccaccccaaa actttcctac cgttagacaa atactggcta    1200
   aatctgacga aaacaaccaa tcaacaattg aatccactgg gaggtatctc taatccactg    1260
   acaaactttg ctaaaacaag aaaaagtggg ggcctccgtt gcggagaaga cgtgcgcagg    1320
   cttaaaaaca caagagaaca cttggaagta ccccagattt ttagcttcct actattctga    1380
   caccccctat tcaagcacga cggtgattga ttcattcaat tttgctgctc caatgatagg    1440
   ataaaccctt ttggacttca atcagacctc tgtcctccat agcaatataa ataccttcta    1500
   gttgccccac ttcctctctc ctgtactgcc ccaatgagtg acttattcaa gttactttct    1560
   ctcttttcct aacaattaaa caagaagctt tattataaca ttaatatact attttataac    1620
   aggattgaaa ttatatttat ctatctaaaa ctaaaattca aa                       1662